**Europäisches Patentamt**

**European Patent Office**

**Office européen des brevets**

(19)

(11) Numéro de publication : **0 402 202 B1**

(12) # FASCICULE DE BREVET EUROPEEN

(45) Date de publication du fascicule du brevet :
**30.03.94 Bulletin 94/13**

(51) Int. Cl.$^5$ : **C07C 15/02,** C07C 2/66, C07C 6/12

(21) Numéro de dépôt : **90401450.3**

(22) Date de dépôt : **31.05.90**

(54) **Procédé de production d'alkylbenzènes utilisant un catalyseur à base de zéolithe Y désaluminée et un catalyseur à base de mordénité désaluminée.**

(30) Priorité : **07.06.89 FR 8907633**

(43) Date de publication de la demande :
**12.12.90 Bulletin 90/50**

(45) Mention de la délivrance du brevet :
**30.03.94 Bulletin 94/13**

(84) Etats contractants désignés :
**BE CH DE ES FR GB IT LI NL SE**

(56) Documents cités :
**EP-A- 0 366 515**
**US-A- 4 169 111**

(73) Titulaire : **INSTITUT FRANCAIS DU PETROLE**
**4, Avenue de Bois Préau**
**F-92502 Rueil-Malmaison (FR)**

(72) Inventeur : **Juguin, Bernard**
**46, avenue du Stade**
**F-92500 Rueil Malmaison (FR)**
Inventeur : **Dufresne, Pierre**
**67, rue Georges Sand**
**F-92500 Rueil Malmaison (FR)**
Inventeur : **Raatz, Francis**
**10 Allée Jacques Prévert**
**F-78260 Acheres (FR)**
Inventeur : **Martino, Germain**
**Batiment Condé, 80 avenue F. Lefebvre**
**F-78300 Poissy (FR)**

EP 0 402 202 B1

Jouve, 18, rue Saint-Denis, 75001 PARIS

## Description

La présente invention concerne un procédé de production d'au moins un alkylbenzène par alkylation du benzène au moyen de mono-oléfine(s). La mono-oléfine ou le mélange de mono-oléfines de départ peut provenir de toute source connue, par exemple d'unités de vapocraquage ou de craquage catalytique.

On a déjà proposé, pour effectuer cette réaction, l'emploi de zéolithes sous forme H, en particulier la ZSM5 (EP - 0104729).

Plus récemment, d'autres zéolithes ont été mises en avant, en particulier certaines mordénites, seules ou en mélange avec de la faujasite (US 3 436 432), éventuellement avec un métal du groupe VIII déposé dessus (US 3 851 004). Des brevets japonais JP 58216128 et JP 58159427, préconisent des mordénites seules, de faible rapport Si/Al (4,5 à 5), contenant des ions métalliques tels que Mg, K, Ca, ou sous forme $H^+$ (JP 041670).

Le document US-A- 4 169 111 décrit un procédé de production d'éthylbenzène dans lequel :

1) on fait réagir du benzène avec de l'éthylène dans un réacteur d'alkylation au contact d'un catalyseur.

2) le produit obtenu est séparé en **4 fractions** :

    a) benzénique

    b) d'éthylbenzène pratiquement pur

    c) de diéthylbenzènes pratiquement pur (DEB)

    d) de triéthylbenzènes (TEB)

3) la fraction (c) est recyclée pour 10-90% (de préférence 20-60%) vers le réacteur d'alkylation.

4) on fait réagir la fraction (d) et la fraction (c) non recyclée (soit préférentiellement 40-80% de la fraction totale DEB) avec du benzène au contact d'un catalyseur.

Plus récemment encore, une demande de brevet français EP-A 366 515 fait état de résultats satisfaisants obtenus en présence de mordénites désaluminées, ayant un rapport atomique Si/Al global compris entre 30 et 80.

L'invention a notamment pour objet d'améliorer le rendement d'alkylation en mono-alkylbenzènes, en réduisant la proportion des poly-alkylbenzènes formés.

Selon le procédé de l'invention, on fait d'abord réagir (première étape ou étape d'alkylation) du benzène avec une charge renfermant au moins une mono-oléfine aliphatique au contact d'au moins un catalyseur à base d'une zéolithe Y désaluminée, de rapport molaire $SiO_2/Al_2O_3$ compris entre 8 et 70, on fractionne le produit de manière à recueillir séparément une première fraction renfermant du benzène non converti et au moins un mono-alkylbenzène et une seconde fraction polyalkylbenzène (c'est-à-dire une fraction renfermant au moins un polyalkylbenzène).

Dans une seconde étape (étape de transalkylation), on fait réagir au moins une partie de ladite fraction polyalkylbenzène avec du benzène, par exemple avec du benzène dont au moins une partie est constituée de benzène non-converti dans la première étape (c'est-à-dire de benzène non-converti provenant de ladite première fraction obtenue dans la première étape) ou par exemple avec du benzène dont au moins une partie n'est pas constituée de benzène non-converti dans la première étape, au contact d'au moins un catalyseur à base d'une mordénite désaluminée de rapport atomique Si/Al global compris entre 30 et 80, et on recueille au moins un mono-alkylbenzène.

Le benzène excédentaire de la première étape qui n'est pas envoyé à la seconde étape est avantageusement recyclé à la première étape, tandis que, après la seconde étape, les polyalkylbenzènes non convertis peuvent être recyclés dans cette même seconde étape.

Ainsi, les mono-alkylbenzènes obtenus par le procédé en deux étapes selon l'invention proviennent, d'une part, de la première fraction obtenue à la première étape (cette fraction pouvant être elle-même fractionnée en benzène non-converti et en au moins un mono-alkylbenzène), et, d'autre part, de la réaction (seconde étape) de la seconde fraction obtenue à la première étape avec du benzène.

La zéolithe Y désaluminée et la mordénite désaluminée sont chacune employées seules ou en mélange avec un liant ou une matrice généralement choisis dans le groupe formé par les argiles, les alumines, la silice, la magnésie, la zircone, l'oxyde de titane, l'oxyde de bore et toute combinaison d'au moins deux des composés précités comme la silice-alumine, la silice-magnésie etc... Toutes les méthodes connues d'agglomération et de mise en forme sont applicables, telles que, par exemple, l'extrusion, le pastillage, la coagulation en gouttes etc...

On utilise ainsi dans le procédé selon 1 invention au moins un catalyseur à base de zéolithe Y désaluminée de rapport molaire $SiO_2/Al_2O_3$ compris entre 8 et 70, contenant généralement 1 à 100 %, de préférence 20 à 98 % et, par exemple, 40 à 98 % en poids de ladite zéolithe Y désaluminée et 0 à 99 %, de préférence 2 à 80 % et, par exemple, 2 à 60 % en poids d'une matrice, et au moins un catalyseur à base de mordénite désaluminée de rapport atomique Si/Al global compris entre 30 et 80, contenant généralement 1 à 100 %, de préférence 20 à 98 % et, par exemple, 40 à 98 % en poids de ladite mordénite désaluminée et 0 à 99 %, de préférence 2

2

à 80 % et, par exemple, 2 à 60 % en poids d'une matrice.

Les zéolithes Y désaluminées et leur préparation sont bien connues. On pourra par exemple se référer au brevet US 4 738 940.

La zéolithe Y utilisée dans la présente invention est une zéolithe acide HY caractérisée par différentes spécifications, dont les méthodes de détermination sont précisées dans la suite du texte : un rapport molaire $SiO_2/Al_2O_3$ compris entre 8 et 70 et, de manière préférée, entre environ 12 et 40 ; une teneur en sodium inférieure à 0,25 % poids déterminée sur la zéolithe calcinée à 1100°C ; un paramètre cristallin a de la maille élémentaire compris entre $24,55 \times 10^{-10}$ et $24,24 \times 10^{-10}$ m et, de manière préférée, entre $24,39 \times 10^{-10}$ et $24,26 \times 10^{-10}$ m ; une capacité $C_{Na}$ de reprise en ions sodium, exprimée en gramme de Na par 100 grammes de zéolithe modifiée, neutralisée puis calcinée, supérieure à environ 0,85 (la capacité $C_{Na}$ de reprise en ions sodium sera définie de manière plus précise au paragraphe suivant) ; une surface spécifique déterminée par la méthode B.E.T. supérieure à environ 400 m²/g et, de préférence, supérieure à 550 m²/g ; une capacité d'adsorption de vapeur d'eau à 25°C, pour une pression partielle de 2,6 torrs, supérieure à environ 6 % ; une répartition poreuse comprenant entre 1 et 20 % et, de préférence, entre 3 et 15 % du volume poreux contenu dans des pores de diamètre situé entre $20 \times 10^{-10}$ et $80 \times 10^{-10}$ m, le reste du volume poreux étant contenu dans les pores de diamètre inférieur à $20 \times 10^{-10}$ m.

Les différentes caractéristiques précédentes peuvent être mesurées par les méthodes suivantes :
- le rapport molaire $SiO_2/Al_2O_3$ peut être mesuré par analyse chimique. Quand les quantités d'aluminium deviennent faibles, par exemple inférieures à 2 %, pour plus de précision, il est opportun d'utiliser une méthode de dosage par spectrométrie d'adsorption atomique ;
- le paramètre de maille peut être calculé à partir du diagramme de diffraction aux rayons X, selon la méthode décrite dans la fiche ASTM D 3.942-80. Il est clair que pour effectuer ce calcul correctement il faut que la cristallinité du produit soit suffisante ;
- la surface spécifique est par exemple déterminée par mesure de l'isotherme d'adsorption d'azote à la température de l'azote liquide et calculée selon la méthode classique B.E.T.. Les échantillons sont prétraités, avant la mesure, à 500°C sous balayage d'azote sec ;
- la distribution poreuse peut être déterminée par la méthode B.J.H., décrite par BARRET, JOYNER et HALENDA dans Journal of Americain Chemical Society, volume 73, page 373 - 1951. Cette méthode est basée sur l'exploitation numérique de l'isotherme de désorption d'azote. La mesure est effectuée avec un appareil CARLO ERBA type SORPTOMATIC Série 1800. Les résultats sont exprimés par les valeurs du volume poreux V en fonction du diamètre des pores D ; la courbe dérivée est également présentée :

$\frac{dV}{dD}$ en fonction de D. Le volume poreux total est défini ici comme le volume d'azote adsorbé à saturation, plus exactement à une pression partielle correspondant à un rapport entre la pression partielle et la pression de vapeur saturante P/Po égal à 0,99 ;
- les pourcentages de reprise en eau (ou capacité d'adsorption de vapeur d'eau) sont par exemple déterminés à l'aide d'un appareillage classique de gravimétrie. L'échantillon est prétraité à 400°C sous vide primaire, puis porté à une température stable de 25°C. On admet ensuite une pression d'eau de 2,6 torrs, ce qui correspond à un rapport P/Po d'environ 0,10 (rapport entre la pression partielle d'eau admise dans l'appareil et la pression de vapeur saturante de l'eau à la température de 25°C) ;
- la capacité d'échange des ions sodium $C_{Na}$ (ou capacité de reprise en ions sodium) peut être déterminée de la manière suivante : un gramme de zéolithe est soumis à trois échanges successifs dans 100 cm³ de solution 0,2M de NaCl, pendant une heure à 20°C sous bonne agitation. Les solutions sont laissées à pH naturel pendant l'échange. En effet, si le pH était réajusté à des valeurs proches de 7, par addition de petites quantités de soude, les taux de sodium échangé seraient supérieurs. Elle est exprimée en gramme de sodium par 100 grammes de zéolithe modifiée.

On a découvert dans la présente invention que les zéolithes Y stabilisées répondant aux spécifications précitées avaient des propriétés remarquables pour la production de mono-alkylbenzènes par alkylation du benzène au moyen de mono-oléfine(s).

Ces zéolithes sont par exemple fabriquées, généralement à partir d'une zéolithe Y-Na, par une combinaison appropriée de deux traitements de base : (a) un traitement hydrothermique qui associe température et pression partielle de vapeur d'eau, et (b) un traitement acide par, de préférence, un acide minéral fort et concentré.

Généralement la zéolithe Y-Na à partir de laquelle on prépare la zéolithe Y utilisée dans l'invention possède un rapport molaire $SiO_2/Al_2O_3$ compris entre environ 4 et 6 ; il conviendra au préalable d'en abaisser la teneur pondérale en sodium à moins de 3 % et, de préférence, à moins de 2,5 % ; la zéolithe Y-Na possède en outre généralement une surface spécifique comprise entre environ 750 et 950 m²/g.

Les mordénites désaluminées et leur préparation sont également bien connues. On pourra par exemple se référer aux brevets européens EP-0084748, 0097552 et 0196965 et à la demande EP-A-366 515. La méthode de désalumination des brevets ou de la demande précités consiste à soumettre la forme H de la mordénite ou un précurseur de forme H (par exemple la forme NH$_4$) à une série de chauffages en atmosphère de vapeur d'eau et de traitements acides. Toutefois, par différence avec les traitements desdits documents précités, on limitera ici le taux de désalumination à un rapport atomique global Si/Al entre 30 et 80. Le brevet EP-0084748, page 4, décrit d'ailleurs des préparations aboutissant à des rapports atomiques Si/Al globaux de 46 et 82 par exemple. On opère de préférence comme suit :

- dans une première étape, on élimine les cations non décomposables, généralement Na$^+$, présents dans la mordénite de départ. Pour ce faire, on pourra procéder à un ou plusieurs échanges dans des solutions diluées d'acides comme HCl ou dans des solutions de NH$_4$+, éventuellement suivis d'un ou plusieurs lavages (à l'eau par exemple). Le point important est que, à l'issue de cette première étape que l'on peut qualifier de décationisation, la quasi-totalité des cations alcalins soit éliminée (teneur en Na par exemple comprise entre 150 et 2000 ppm en poids et, de préférence, entre 300 et 1200 ppm en poids) et le solide obtenu soit une forme H ou un précurseur de forme H (exemple NH$_4$+) non désaluminée substantiellement (taux de désalumination généralement inférieur à 10 % et, de préférence, à 5 %). De manière préférée, on choisira, comme précurseur de forme H, la forme NH$_4$+ ;
- dans une deuxième étape, la forme H ou le précurseur de forme H est soumis à un traitement sous vapeur d'eau à une température supérieure à 450°C, par exemple comprise entre 450 et 650°C et, de préférence, entre 550 et 600°C. La teneur en eau (en volume) de l'atmosphère de calcination sera avantageusement supérieure à 20 % et, de manière préférée, à 40 % ;
- l'attaque acide constitue la troisième étape de la préparation des catalyseurs. Pour des rapports atomiques Si/Al de charpente (du solide après calcination) jusqu'à 50 environ, on utilisera de préférence des concentrations de solutions acides (HCl, H$_2$SO$_4$, HNO$_3$, etc...) comprises entre 0,5 et 5 N et, de préférence, entre 1 et 4N. Pour des rapports atomiques Si/Al de charpente supérieurs, on utilisera des concentrations de solutions acides comprises entre 5 et 20 N et, de préférence, entre 7 et 12 N (les rapports atomiques Si/Al de charpente peuvent être déterminés par spectroscopie infra-rouge pour des rapports compris entre 10 et 50 et par RMN du $^{29}$Si pour des rapports supérieurs). Par ailleurs, pour atteindre des rapports atomiques Si/Al élevés, c'est-à-dire supérieurs à environ 50 et plus spécifiquement supérieurs à environ 60, on pourra recourir avantageusement à plusieurs cycles calcination sous vapeur d'eau-attaque acide.

Les solides ainsi préparés ont avantageusement des rapports atomiques Si/Al globaux de 30 à 80 ; ils ont un volume de maille élémentaire situé entre 2,755 et 2,730 nm$^3$ (1 nm = 10$^{-9}$ m) et, de préférence, entre 2,745 et 2,735 nm$^3$ ; ils ont de préférence une force acide suffisante pour que les Al-OH structuraux intéragissent avec une base faible comme l'éthylène (mesure infra-rouge à 77 K) ou un composé à caractère faiblement acide comme H$_2$S (mesure infra-rouge à 25°C). Ces solides doivent, en outre, être de préférence exempts d'espèces cationiques extra-réseau que l'on peut détecter par un signal fin (largeur à mi-hauteur inférieure à 5 ppm et, de préférence, inférieure à 2 ppm) situé à 0 ppm (référence Al(H$_2$O)$_6$ $^{3+}$) sur un spectre de RMN de $^{27}$Al, mesuré avec la technique de rotation de l'angle magique.

La réaction dite d'alkylation est habituellement effectuée en phase liquide, en phase supercritique ou en phase gazeuse, en présence d'au moins un catalyseur à base de la zéolithe Y désaluminée définie plus haut, disposé en lit fixe, à une température d'environ 50 à 450°C (de préférence d'environ 100 à 350°C), sous une pression de 1 à 10 MPa (de préférence de 2 à 7 MPa), avec un débit d'hydrocarbures liquides (vitesse spatiale) d'environ 0,5 à 50 volumes par volume de catalyseur et par heure, et avec un rapport molaire benzène/mono-oléfines compris entre 1 et 20 (de préférence, entre 3 et 7 pour le propène et entre 7 et 12 pour l'éthylène).

La réaction dite de transalkylation des polyalkylbenzènes formés au cours de l'étape d'alkylation est habituellement effectuée en présence d'au moins un catalyseur à base de la mordénite désaluminée définie plus haut, disposé en lit fixe, à une température d'environ 200 à 500°C (de préférence d'environ 280 à 400°C), sous une pression de 2 à 10 MPa (de préférence de 2,5 à 7 MPa), avec un débit d'hydrocarbures liquides (vitesse spatiale) d'environ 0,5 à 5 volumes par volume de catalyseur et par heure, et avec un rapport molaire benzène/polyalkylbenzènes de 2 à 50.

Bien que l'on puisse mettre en oeuvre l'invention avec un mélange de mono-oléfines, par exemple un effluent de craquage catalytique, on préfère, afin de faciliter le fractionnement entre mono-alkylbenzènes et polyalkylbenzènes, opérer avec une seule mono-oléfine, par exemple l'éthylène, le propylène, un n-butène ou l'isobutène, plus généralement avec une mono-oléfine aliphatique ayant par exemple de 2 à 20 atomes de carbone.

Le traitement de mélanges de mono-oléfines renfermant du monoxyde de carbone, par exemple 0,1 à 2,0 % en poids, ce qui est souvent le cas des effluents de cracking, présente un intérêt particulier. En effet la présence du monoxyde de carbone n'a pas d'effet néfaste, contrairement à ce qui est observé avec les catalyseurs

EP 0 402 202 B1

conventionnels renfermant du nickel.

Pour simplifier la mise en oeuvre de l'invention, on peut utiliser une zone de distillation unique pour les effluents des deux étapes du procédé (alkylation et transalkylation) selon toute technique bien connue de l'homme du métier.

Les exemples suivants illustrent la présente invention sans en limiter la portée.

EXEMPLE 1 : Préparation d'un catalyseur A.

On utilise comme matière première une zéolithe NaY de formule $Na\,AlO_2\,(SiO_2)_{2,5}$.

Cette zéolithe présente les caractéristiques suivantes :
- rapport molaire $SiO_2/Al_2O_3$     : 5
- paramètre cristallin a     : 24,69 x $10^{-10}$ m
- capacité d'adsorption de vapeur d'eau à 25°C (à P/Po = 0,1)     : 26 %
- surface spécifique     : 880 m$^2$/g.

Elle est soumise à cinq échanges consécutifs dans des solutions de nitrate d'ammonium de concentration 2M, à une température de 95°C, pendant un temps de 1,5 heure, et avec un rapport volume de solution sur poids de zéolithe égal 8 cm$^3$/g. Le taux de sodium de la zéolithe $NaNH_4Y$ obtenu est 0,95 %. Ce produit est ensuite introduit rapidement dans un four préchauffé à 770°C et laissé pendant 4 heures en atmosphère statique (traitement stabilisant). La zéolithe est soumise ensuite à deux échanges par des solutions de nitrate d'ammonium de concentration 2 M de manière à ce que sa teneur en sodium descende à 0,2 % poids. Son rapport molaire $SiO_2/Al_2O_3$ est alors de 6,3, son paramètre cristallin a de la maille élémentaire de 24,38x$10^{-10}$ m, sa surface spécifique de 625 m$^2$/g, sa capacité de reprise en eau de 11,3 % (à P/Po = 0,1), sa capacité de reprise en ions sodium de 2 et 11 % de son volume poreux est contenu dans des pores de diamètre situé entre et 20x$10^{-10}$ et 80x$10^{-10}$ m, le reste de son volume poreux étant contenu dans des pores de diamètre inférieur à 20x$10^{-10}$ m. La zéolithe ainsi obtenue est mise en forme par extrusion avec de l'alumine. Les extrudés obtenus sont ensuite séchés puis calcinés vers 500°C environ. On obtient alors un catalyseur A à base de ladite zéolithe, contenant 80 % en poids de ladite zéolithe et 20 % en poids d'alumine.

EXEMPLE 2 : Préparation d'un catalyseur B.

La zéolithe NaY de départ est soumise aux mêmes cinq échanges et au même traitement stabilisant que dans l'exemple 1. Après la stabilisation, au lieu de faire des échanges avec des ions ammonium, on procède à un traitement acide dans les conditions suivantes : le rapport entre le volume de solution d'acide nitrique 2N et le poids de solide est de 6 cm$^3$/g, la température est de 95°C et la durée du traitement de 3 heures. Ensuite un autre traitement dans les mêmes conditions est effectué, mais avec une solution d'acide nitrique 0,3N.

La zéolithe obtenue possède une teneur pondérale en sodium de 0,2 %, un rapport molaire $SiO_2/Al_2O_3$ de 18, un paramètre cristallin a de la maille élémentaire de 24,32x$10^{-10}$ m, une surface spécifique de 805 m$^2$/g, une capacité de reprise en eau de 13,7 % (à P/Po = 0,1), une capacité de reprise en ions sodium de 2 et 9 % de son volume poreux est contenu dans des pores de diamètre situé entre 20x$10^{-10}$ et 80x$10^{-10}$ m, le reste de son volume poreux étant contenu dans des pores de diamètre inférieur à 20x$10^{-10}$ m.

Les étapes de mise en forme, de séchage et de calcination sont effectuées dans les mêmes conditions que celles décrites dans l'exemple 1, de manière à obtenir un catalyseur B à base de ladite zéolithe, contenant 80 % en poids de ladite zéolithe et 20 % en poids d'alumine.

EXEMPLE 3 : Préparation d'un catalyseur C.

La zéolithe NaY de départ est soumise aux mêmes cinq échanges et au même traitement stabilisant que dans l'exemple 1.

Après la stabilisation, au lieu de faire des échanges avec des ions ammonium, on procède à un traitement acide à l'aide d'une solution d'acide nitrique 2N dans les conditions suivantes : le rapport entre le volume de solution d'acide nitrique 2N et le poids de solide est de 6 cm$^3$/g, la température est de 95°C et la durée du traitement de 3 heures. Ensuite un autre traitement dans les mêmes conditions est effectué, mais avec une solution d'acide nitrique 3N.

La zéolithe obtenue possède une teneur pondérale en sodium de 0,2 %, un rapport molaire $SiO_2/Al_2O_3$ de 75, un paramètre cristallin a de la maille élémentaire de 24,28x$10^{-10}$ m, une surface spécifique de 795 m$^2$/g, une capacité de reprise en eau de 13,5 % (à P/Po = 0,1), une capacité de reprise en ions sodium de 2 et 10 % de son volume poreux est contenu dans des pores de diamètre situé entre 20x$10^{-10}$ m et 80x$10^{-10}$ m, le reste de son volume poreux étant contenu dans des pores de diamètre inférieur à 20x$10^{-10}$ m.

Les étapes de mise en forme, de séchage et de calcination sont effectuées dans les mêmes conditions que celles décrites dans l'exemple 1, de manière à obtenir un catalyseur C à base de ladite zéolithe, contenant 80 % en poids de ladite zéolithe et 20 % en poids d'alumine.

EXEMPLE 4 : Préparation d'un catalyseur D.

La matière première utilisée pour préparer ce catalyseur est une mordénite petits pores de la Société Chimique de la Grande Paroisse, référencée Alite 150 ; sa formule chimique à l'état anhydre est Na, $AlO_2(SiO_2)_{5,5}$ et sa teneur pondérale en sodium de 5,3 %. 500 grammes de cette poudre sont plongés dans une solution 2 M de nitrate d'ammonium, et la suspension est portée à 95°C pendant 2 heures. Le volume de la solution de nitrate d'ammonium engagée est égal à 4 fois le poids de mordénite sèche (V/P = 4 g/cm³). Cette opération d'échange cationique est recommencée 3 fois. Après le 3ème échange, le produit est lavé à l'eau à 20°C pendant 20 minutes, avec un rapport V/P égal à 4 g/cm³. La teneur en sodium exprimée en pourcentage en poids par rapport à la mordénite sèche n'est plus que de 0,1 %. Le produit est ensuite filtré, et soumis une calcination sous vapeur d'eau en atmosphère confinée ("self-steaming") à 560°C pendant 2 heures. La teneur en eau (en volume) de l'atmosphère de calcination est de l'ordre de 90 %. La cristallinité de ce solide, après cette étape de calcination, est supérieure ou égale à 90 % ; son rapport atomique Si/Al de charpente est égal à 49.

On procède ensuite sur ce solide à une attaque acide à l'aide d'une solution d'acide nitrique 3,5N. Au cours de l'attaque acide, le solide est ainsi porté à reflux dans la solution d'acide nitrique pendant 2 heures, avec un rapport V/P égal à 8 g/cm³. Le produit est ensuite filtré, puis lavé abondamment à l'eau distillée.

La mordénite obtenue possède un rapport atomique Si/Al global de 49 ; son volume de maille élémentaire est égal à 2,74 nm³.

Elle est ensuite mise en forme par malaxage avec de l'alumine, puis par passage au travers d'une filière. Les extrudés obtenus, de diamètre 1,2 mm, sont ensuite séchés, puis calcinés entre 150 et 500°C par palier d'une heure environ. On obtient alors un catalyseur D à base de ladite mordénite, contenant 80 % en poids de ladite mordénite et 20 % en poids d'alumine.

EXEMPLE 5

Les trois catalyseurs A, B et C préparés dans les exemple 1, 2 et 3 sont chacun testés en alkylation du benzène par l'éthylène dans les conditions opérations suivantes (dans un réacteur dit "réacteur d'alkylation") :
- température : 270°C,
- pression : 3 MPa,
- débit horaire pondéral de benzène égal à 2 fois le poids du catalyseur,
- rapport molaire benzène/éthylène : 9.
La charge a la composition pondérale suivante :
- éthylène : 3,73 %
- benzène : 96,27 %.

A la sortie du réacteur, les produits obtenus ont la composition pondérale présentée dans le tableau 1. On peut constater qu'il est préférable de travailler, lors de l'étape d'alkylation, avec les catalyseurs préconisés par l'invention, c'est-à-dire qu'il faut utiliser des zéolithes Y désaluminées dont le rapport molaire $SiO_2/Al_2O_3$ est compris entre 8 et 70 (cas du catalyseur B). En effet, les zéolithes Y ayant un rapport molaire $SiO_2/Al_2O_3$ inférieur à 8 (cas du catalyseur A) sont légèrement plus actives, mais nettement moins sélectives et peu stables. En revanche, les zéolithe Y ayant un rapport molaire $SiO_2/Al_2O_3$ supérieur à 70 (cas du catalyseur C) sont un peu plus sélectives, mais beaucoup moins actives, ce qui conduirait industriellement à travailler en leur présence soit à température nettement plus élevée conduisant à des durées de cycle plus faibles, soit à des vitesses spatiales plus faibles ce qui serait néfaste pour l'économie du procédé.

TABLEAU 1

| CATALYSEURS CONSTITUANTS (% poids) | A | B | C |
|---|---|---|---|
| | CHARGE | | |
| Ethylène | 3,73 | - | 0,01 | 1,42 |
| Benzène | 96,27 | 87,12 | 86,28 | 90,02 |
| Toluène | - | 0,03 | - | - |
| Ethylbenzène | - | 11,05 | 13,10 | 8,25 |
| Méthyléthylbenzènes | - | 0,04 | - | - |
| Diéthylbenzènes | - | 1,17 | 0,55 | 0,29 |
| Méthyldiéthylbenzènes | - | 0,03 | - | - |
| Triéthylbenzènes | - | 0,31 | 0,04 | 0,02 |
| HC aromatiques lourds | - | 0,25 | 0,02 | - |
| | | 100 | 100 | 100 |
| TAUX DE CONVERSION PAR PASSE | | | |
| Ethylène | | 100 % | 99,7 % | 61,9 % |
| Benzène | | 9,5 % | 10,4 % | 6,5 % |
| SELECTIVITES | | | |
| Ethylbenzène ─────────────── x 100 Ethylène transformé | | 78,3 % | 93,0 % | 94,3 % |
| Ethylbenzène ─────────────── x 100 Benzène transformé | | 88,8 % | 96,5 % | 97,2 % |

Après réaction, le produit obtenu en présence du catalyseur B est fractionné par distillation, et deux fractions sont recueillies :
- une fraction point initial - 140°C contenant le benzène non converti et l'éthylbenzène,
- une fraction de point d'ébullition supérieur à 140°C contenant les polyéthylbenzènes et les hydrocarbures (ou HC) aromatiques lourds.

Cette fraction de point d'ébullition supérieur à 140°C subit ensuite un traitement de transalkylation en présence du catalyseur D (préparé dans l'exemple 4) dans les conditions opératoires suivantes (dans un réacteur dit "réacteur de transalkylation") :

- température : 360°C,
- pression : 3 MPa,
- débit pondéral de charge (polyéthylbenzènes + hydrocarbures aromatiques lourds + benzène additionnel qui provient du produit de la réaction d'alkylation) égal à 1 fois le poids du catalyseur,
- rapport molaire benzène/polyéthylbenzènes : 16.

Les résultats obtenus (par le traitement de transalkylation) sont présentés dans le tableau 2.

Les résultats d'ensemble combinant les deux étapes d'alkylation d'une part et de transalkylation d'autre part sont les suivants :

- taux de transformation du benzène : 10,7 %
- taux de transformation de l'éthylène : 99,7 %
- sélectivité en éthylbenzène par rapport à l'éthylène transformé : 98,7 %
- sélectivité en éthylbenzène par rapport au benzène transformé : 99,4 %.

En ce qui concerne la mise en oeuvre industrielle de ce type de procédé, les diéthylbenzènes et les triéthylbenzènes non transformés après l'étape de transalkylation sont recyclés à extinction dans le réacteur de transalkylation ; dans ce cas, en utilisant les catalyseurs B (dans l'étape d'alkylation) et D (dans l'étape de transalkylation), les performances ultimes sont les suivantes :

1) Taux de conversion :
   - benzène : 10,7 %
   - éthylène : 99,7 %.
2) Sélectivités :
   - éthylbenzène par rapport au benzène transformé : 99,4 %
   - éthylbenzène par rapport à l'éthylène transformé : 99,6 %.

## TABLEAU 2

| CONSTITUANTS | CHARGE | PRODUIT |
|---|---|---|
| Benzène | 5,58 | 5,29 |
| Ethylbenzène | - | 0,79 |
| Diéthylbenzènes | 0,55 | 0,07 |
| Triéthylbenzènes | 0,04 | 0,01 |
| HC aromatiques lourds | 0,02 | 0,03 |
| | 6,19 | 6,19 |
| TAUX DE CONVERSION PAR PASSE | | |
| Benzène | | 5,2 % |
| Diéthylbenzènes | | 87,3 % |
| Triéthylbenzènes | | 75,0 % |
| SELECTIVITE | | |
| $\dfrac{\text{Ethylbenzène}}{(\text{Diéthylbenzènes} + \text{triéthylbenzènes}) \text{transformés}} \times 100$ | | 98,8 % |

## EXEMPLE 6

On opère dans les mêmes conditions que celles de l'exemple 5, en utilisant notamment le catalyseur B dans la section d'alkylation et le catalyseur D dans la section de transalkylation, tout en faisant varier le rapport molaire benzène/éthylène 1 entrée du réacteur d'alkylation, afin de montrer l'importance de ce paramètre sur l'activité et surtout la sélectivité des catalyseurs. Les résultats d'ensemble combinant les deux étapes d'alkylation d'une part et de transalkylation d'autre part sont présentés dans le tableau 3.

## TABLEAU 3

| BENZENE molaire | | | |
|---|---|---|---|
| ETHYLENE | 5,1 | 9,3 | 13,7 |
| RESULTATS | | | |
| TAUX DE CONVERSION | | | |
| ETHYLENE | 98,2 % | 99,7 % | 100 % |
| SELECTIVITES | | | |
| 1) Ethylbenzène formé par rapport au benzène transformé | 97,8 % | 99,4 % | 99,6 % |
| 2) Ethylbenzène formé par rapport à l'éthylène transformé | 93,6 % | 98,7 % | 99,5 % |

On peut constater la grande influence de ce paramètre sur la sélectivité des catalyseurs ; comme préconisé dans le cadre de l'invention, il est préférable de travailler avec un rapport molaire benzène/éthylène compris entre 7 et 12. En effet, pour des rapports molaires benzène/éthylène inférieurs à 7, la sélectivité est relativement basse. Pour des rapports molaires benzène/éthylène supérieurs à 12, si la sélectivité est bonne, il est nécessaire d'adopter des taux de recyclage de benzène très élevés, ce qui grève fortement l'économie globale du procédé.

EXEMPLE 8

Le catalyseur B, préparé dans l'exemple 3, est testé en alkylation du benzène par le propène dans les conditions opératoires suivantes (dans un réacteur dit "réacteur d'alkylation") :
- température     : 220°C,
- pression     : 3 MPa,
- débit horaire pondéral de benzène égal à 2 fois le poids du catalyseur,
- rapport molaire benzène/propène    : 5.
La charge a la composition pondérale suivante :
- propène    : 9,72 %
- benzène    : 90,28 %.
A la sortie du réacteur, le produit obtenu a la composition pondérale suivante :

- propène    :   -
- benzène    : 72,58 %
- cumène    : 26,74 %
- diisopropylbenzènes    : 0,60 %
- triisopropylbenzènes    : 0,06 %
- HC aromatiques lourds    : 0,02 %
                  100 %

Les taux de conversion sont donc les suivants :
- benzène          : 19,6 %
- propène          : 100%.

Les sélectivités sont respectivement les suivantes :
- cumène par rapport au benzène transformé          : 98,2 %
- cumène par rapport au propène transformé          : 96,3 %.

Après réaction, le produit obtenu en présence du catalyseur B est fractionné par distillation, et deux fractions sont recueillies :
- une fraction point initial - 160°C contenant le benzène non-converti et le cumène,
- une fraction de point d'ébullition supérieur à 160°C contenant les polyisopropylbenzènes et les hydrocarbures aromatiques lourds.

Cette fraction de point d'ébullition supérieur à 160°C subit ensuite un traitement de transalkylation en présence du catalyseur D (préparé dans l'exemple 4), dans les conditions opératoires suivantes (dans un réacteur dit "réacteur de transalkylation) :
- température          : 330°C
- pression          : 3 MPa
- débit pondéral de charge (polyisopropylbenzènes + hydrocarbures lourds + benzène additionnel qui provient du produit de la réaction d'alkylation) égal à 1 fois le poids du catalyseur
- rapport molaire benzène/polyisopropylbenzènes = 10.

La charge (pour le traitement de transalkylation) a ainsi la composition pondérale suivante :

```
- benzène                    :  3,19
- diisopropylbenzènes        :  0,60
- triisopropylbenzènes       :  0,06
- HC aromatiques lourds      :  0,02
                                ─────
                                 3,87
```

A la sortie du réacteur de transalkylation, le produit obtenu a la composition pondérale suivante :

```
- benzène                    :  2,91
- cumène                     :  0,83
- diisopropylbenzènes        :  0,07
- triisopropylbenzènes       :  0,02
- HC aromatiques lourds      :  0,04
                                ─────
                                 3,87
```

Les résultats d'ensemble combinant les deux étapes d'alkylation d'une part et de transalkylation d'autre part sont les suivants :
- taux de transformation du benzène          : 19,9 %
- taux de transformation du propène          : 100 %
- sélectivité en cumène par rapport au propène transformé          : 99,3 %
- sélectivité en cumène par rapport au benzène transformé          : 99,7 %.

**Revendications**

1.  Procédé de production d'au moins un alkylbenzène dans lequel on fait réagir du benzène avec une charge renfermant au moins une mono-oléfine aliphatique en présence d'au moins un catalyseur à base d'une zéolithe Y désaluminée de rapport molaire $SiO_2/Al_2O_3$ compris entre 8 et 70, procédé caractérisé en ce qu'on fractionne le produit obtenu de manière à recueillir séparément une première fraction renfermant du benzène non-converti et au moins un mono-alkylbenzène et une seconde fraction renfermant au moins un polyalkylbenzène, on fait réagir au moins une partie de ladite seconde fraction avec du benzène au contact d'au moins un catalyseur à base d'une mordénite désaluminée de rapport atomique Si/Al global compris entre 30 et 80, et on recueille au moins un mono-alkylbenzène.

2. Procédé selon la revendication 1 dans lequel la mordénite désaluminée présente un rapport atomique Si/Al compris entre 49 et 80.

3. Procédé selon les revendications 1 ou 2 dans lequel au moins une partie du benzène avec lequel on fait réagir au moins une partie de ladite seconde fraction est constituée de benzène non-converti provenant de ladite première fraction.

4. Procédé selon l'une des revendications 1 à 3 dans lequel ladite zéolithe Y désaluminée possède un rapport molaire $SiO_2/Al_2O_3$ compris entre environ 12 et 40.

5. Procédé selon l'une des revendications 1 à 4 dans lequel chaque catalyseur contient en outre une matrice.

6. Procédé selon l'une des revendications 1 à 5 dans lequel chaque catalyseur est disposé en lit fixe.

7. Procédé selon l'une des revendications 1 à 6 dans lequel les polyalkylbenzènes non-convertis lors de l'étape de réaction du benzène avec au moins une partie de ladite seconde fraction sont recyclés dans cette étape.

8. Procédé selon l'une des revendications 1 à 7 dans lequel ladite charge renferme 0,1 à 2,0 % en poids de monoxyde de carbone.

9. Procédé selon l'une des revendications 1 à 8 dans lequel ladite charge est un effluent de craquage catalytique.

10. Procédé de production d'éthylbenzène selon l'une des revendications 1 à 9 dans lequel, lors de la réaction du benzène avec une charge renfermant de l'éthylène, le rapport molaire benzène/éthylène est compris entre 7 et 12.

11. Procédé de production de cumène selon l'une des revendications 1 à 9 dans lequel, lors de la réaction du benzène avec une charge renfermant du propène, le rapport molaire benzène/propène est compris entre 3 et 7.


**Patentansprüche**

1. Verfahren zur Herstellung wenigstens eines Alkylbenzols, bei dem man Benzol mit einer wenigstens ein aliphatisches Mono-Olefin enthaltenden Charge, in Anwesenheit wenigstens eines Katalysators auf der Basis eines entaluminierten Zeoliths vom Y-Typ mit einem Molverhältntis $SiO_2/Al_2O_3$, das zwischen 8 und 70 liegt, ragieren läßt, **dadurch gekennzeichnet,** daß man das erhaltene Produkt derart fraktioniert, daß man getrennt eine nicht umgesetztes Benzol und wenigstens ein Monoalkylbenzol enthaltende erste Fraktion und eine zweite Fraktion gewinnt, die wenigstens ein Polyalkylbenzol enthält, wenigstens einen Teil dieser zweiten Fraktion mit Benzol im Kontakt mit wenigstens einem Katalysator auf der Basis eines entaluminierten Mordenits mit einem Atomgesamtverhältnis Si/Al, das zwischen 30 und 80 liegt, reagieren läßt und wenigstens ein Monoalkylbenzol gewinnt.

2. Verfahren nach Anspruch 1, bei dem der entaluminierte Mordenit ein Atomverhältnis Si/Al zwischen 49 und 80 aufweist.

3. Verfahren nach Anspruch 1, bei dem wenigstens ein Teil des Benzols, mit dem man wenigstens einen Teil dieser zweiten Fraktion reagieren läßt, gebildet wird aus nicht umgesetztem Benzol, das aus dieser ersten Fraktion stammt.

4. Verfahren nach einem der Ansprüche 1 und 2, bei dem dieser entaluminierte Zeolith vom Y-Typ ein Molverhältnis $SiO_2/Al_2O_3$ zwischen etwa 12 und 40 besitzt.

**5.** Verfahren nach einem der Ansprüche 1 bis 3, bei dem jeder Katalysator im übrigen eine Matrix enthält.

**6.** Verfahren nach einem der Ansprüche 1 bis 4, bei dem jeder Katalysator als Festbett angeordnet ist.

**7.** Verfahren nach einem der Ansprüche 1 bis 5, bei dem die in der Stufe der Umsetzung des Benzols mit wenigstens einem Teil dieser zweiten Fraktion nicht umgesetzten Polyalkylbenzole in diese Stufe rezyklisiert werden.

**8.** Verfahren nach einem der Ansprüche 1 bis 6, bei dem diese Charge 0,1 bis 2,0 Gew.-% Kohlenmonoxid enthält.

**9.** Verfahren nach einem der Ansprüche 1 bis 7, bei dem diese Charge ein katalytischer Krackungs-Abstrom ist.

**10.** Verfahren zur Herstellung von Ethylbenzol nach einem der Ansprüche 1 bis s, bei dem während des Reaktion des Benzols mit einer Ethylen enthaltenden charge das Molverhältnis Benzol/Ethylen zwischen 7 und 12 liegt.

**11.** Verfahren zur Kerstellung von Cumol nach einem der Ansprüche 1 bis 8, bei dem während der Reaktion des Benzols mit einer Propen enthaltenden Charge das Molverhältnis Benzol/Propen zwischen 3 und 7 liegt.


**Claims**

**1.** A process for producing at least one alkylbenzene wherein benzene is reacted with a charge comprising at least one aliphatic mono-olefin in the presence of at least one catalyst based on a dealuminized Y zeolite with a molar ratio $SiO_2/Al_2O_3$ ranging from 8 to 70, process characterized in that the product obtained is fractionated in order to separately recover a first fraction containing non converted benzene and at least one mono-alkylbenzene and a second fraction containing at least one polyalkylbenzene, at least part of said second fraction is reacted with benzene in contact with at least one catalyst based on a dealuminized mordenite with a total atomic ratio Si/Al ranging from 30 to 80, and at least one mono-alkylbenzene is recovered.

**2.** A process according to claim 1, wherein the dealuminized mordenite shows a atomic ratio i/Al ranging from 49 to 80.

**3.** A process according to claims 1 or 2, wherein at least part of the benzene which at least part of said second fraction is reacted with consists of non converted benzene coming from said first fraction.

**4.** A process according to any one of claims 1 to 3 wherein said dealuminized Y zeolite shows a molar ratio $SiO_2/Al_2O_3$ ranging from about 12 to 40.

**5.** A process according to any one of claims 1 to 4 wherein each catalyst also contains a matrix.

**6.** A process according to any one of claims 1 to 5 wherein each catalyst is arranged in a fixed bed.

**7.** A process according to any one of claims 1 to 6 wherein the polyalkylbenzenes which have not been converted during the stage of the reaction of benzene with at least part of said second fraction are recycled in this stage.

**8.** A process according to any one of claims 1 to 7 wherein said charge comprises 0.1 to 2.0 % by weight of carbon oxide.

**9.** A process according to any one of claims 1 to 8 wherein said charge is a catalytic cracking effluent.

**10.** A process for producing ethylbenzene according to any one of claims 1 to 9 wherein, during the reaction of benzene with a charge containing ethylene, the molar ratio benzene/ethylene ranges from 7 to 12.

**11.** A process for producing cumene according to any one of claims 1 to 9 wherein, during the reaction of benzene with a charge containing propene, the molar ratio benzene/propene ranges from 3 to 7.